# EUROPÄISCHE PATENTANMELDUNG

(11) **EP 0 663 194 A1**
(43) Veröffentlichungstag der Anmeldung: **19.07.1995**
(21) Anmeldenummer: 95100267.4
(22) Anmeldetag: 10.01.1995
(51) Int. Cl.: A61F 2/36

(54) **Alloplastischer Knochenersatz, insbesondere Gelenkendoprothese, enthaltend eine Konussteckverbindung**

(30) Priorität: 17.01.1994 DE 9400690 U
(71) Anmelder: Waldemar Link (GmbH & Co.), D-22339 Hamburg (DE)
(72) Erfinder: Link, Helmut D., D 22397 Hamburg (DE)
(74) Vertreter: Glawe, Delfs, Moll & Partner

(57) **Zusammenfassung**

Ein alloplastischer Knochenersatz, insbesondere für eine Gelenkendoprothese, weist eine Konussteckverbindung mit zwei Komponenten (1, 3) auf, die durch Korrosion gefährdet ist. Damit Korrosionsprodukte nicht nach außen gelangen können, sind die zusammenwirkenden Konusflächen (2, 4) der Konussteckverbindung nach außen hin abgedichtet.

## Beschreibung

Es hat sich gezeigt (Saikko u.a. "Wear of the polyethylene acetabular cup", Acta Orthop Scand 1993, S. 391-402), daß die Konusflächen, die zur Konusverbindung eines Hüftprothesenkopfs mit dem Hüftprothesenschaft dienen, im Gebrauch einer u.U. beträchtlichen Korrosion unterliegen. Man hat dies dadurch zu verhindern gesucht (EP-A 597553), daß man eine Folie aus thermoplastischem Kunststoff zwischen die Konusflächen eingefügt hat, die durch Erhitzung mit den Konusflächen fest verbunden wird. Da die Teile jedoch häufig erst während der Operation miteinander verbunden werden können, eignet sich dieses bekannte Verfahren lediglich für die fabrikmäßige Herstellung. Denselben Nachteil hat das Einspritzen einer thermoplastischen Trennschicht aus dem flüssigen Zustand (EP-A 193681). Gemäß einem anderen Vorschlag (EP-A 562782) wird eine metallische Konushülse zwischen die zu verbindenden Konusflächen eingesetzt, die eine elektrisch nicht gleitende, oxydische Oberflächenschicht aufweist. Dies ist aufwendig, wenn die Oberflächenschicht so dick sein soll, daß sie keinesfalls unter der mechanischen Beanspruchung unter Wiederherstellung der Leitfähigkeit verletzt wird.

Die Erfindung sucht nach einer Lösung zur Vermeidung der Nachteile der bekannten Vorrichtungen. Unter Beibehaltung des direkten Kontakts zwischen den korrosionsgefährdeten Konusflächen schlägt sie vor, diese nach außen hin abzudichten. Eine Wirkung dieser Maßnahme besteht darin, daß der Körperflüssigkeit der Zutritt zu den Konusflächen verwehrt wird, wodurch die Korrosion bis zu einem gewissen Grade zurückgedrängt wird. Wichtiger noch ist die Wirkung, daß etwaige Korrosionsprodukte innerhalb des Verbindungsbereichs zurückgehalten werden, um nicht störend vagabundieren und dann beispielsweise als abriebfördernde Partikeln in künstliche oder natürliche Gelenke gelangen zu können.

Zweckmäßigerweise ist in einem der beiden Verbindungsteile - im allgemeinen ist dies der weibliche Verbindungsteil - eine Nut vorgesehen, die nahe dem äußeren Ende ihrer mit dem anderen Teil zusammenwirkenden Konusfläche angeordnet ist und einen Dichtring enthält. Der Dichtring ist vorzugsweise weichelastisch. Jedoch kann es sich auch um ein Material handeln, das sich auf Dauer plastisch so verformt, daß es mit den gegenseitig abzudichtenden Flächen eine feste und dichte Verbindung eingeht.

Die Erfindung wird im folgenden näher und Bezugnahme auf die Zeichnung erläutert, die zwei vorteilhafte Ausführungsbeispiele veranschaulicht, nämlich in
- Fig. 1: eine Ausführung mit innerhalb der weiblichen Konusfläche gelegener Dichtungsnut und
- Fig. 2: eine Ausführung mit vorgesetzter Dichtung.

In beiden Ausführungen ist der proximale Teil 1 einer femoralen Hüftprothese mit einem männlichen Verbindungskonus 2 versehen, auf den eine Gelenkkugel 3 aufgesetzt ist. Teil 1 besteht vorzugsweise aus Metall, während der Gelenkkopf 3 vorzugsweise aus Keramikmaterial, unter Umständen aber gleichfalls aus Metall besteht. Der Gelenkkopf 3 enthält eine zu dem Verbindungskonus 2 passende weibliche Konusbohrung 4.

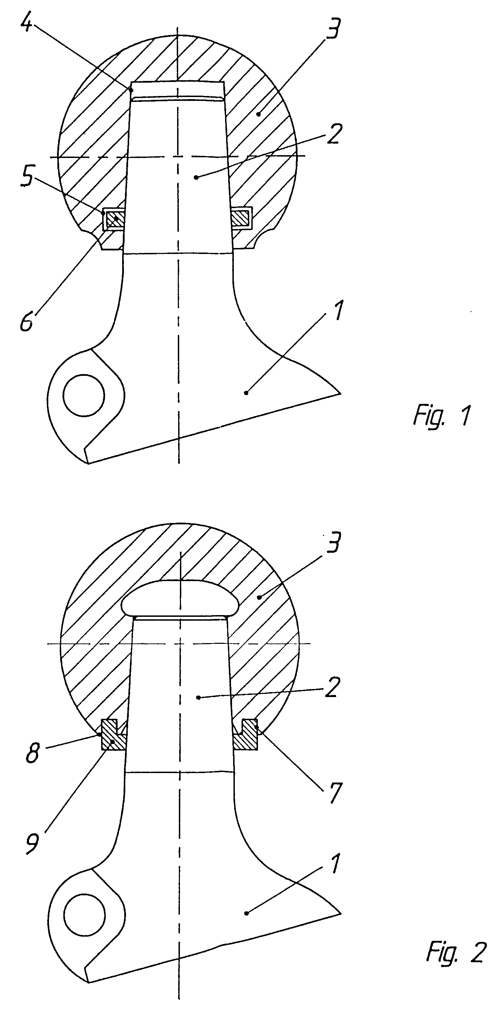

In der Ausführung gemäß Fig. 1 ist nahe dem offenen Ende in die weibliche Konusverbindungsfläche 4 eine Nut 5 zur Aufnahme eines Dichtungsrings 6 eingeschnitten, der eine dichte Verbindung zwischen den beiden Teilen schafft.

Im Ausführungsbeispiel gemäß Fig. 2 ist stirnseitig in den Gelenkkopf 3 eine Nut 7 eingeschnitten, in die der achsparallel verlaufende Rand 8 eines im Längsschnitt winkelförmigen Rings 9 dicht eingepaßt ist, dessen anderer Schenkel sich dichtend an die Oberfläche des Verbindungskonus 2 anlegt. Der Ring wird in der dargestellten Stellung dadurch gehalten, daß sein am Konus anliegender Innenumfang diesen stramm umschließt.

Vorzugsweise ist die Abdichtung flüssigkeitsdicht ausgeführt, so daß Körperflüssigkeit nicht in den Verbindungsbereich hineingelangen kann. Jedoch genügt nach der Erfindung ggf. auch eine Abdichtung, die zwar nicht flüssigkeitsdicht ist, aber den Austritt von Korrosionspartikeln aus dem Verbindungsbereich nach außen verhindert.

Der Dichtring 6 bzw. 9 soll so beschaffen sein, daß er beim Aufschieben keine oder keine wesentliche Rückstellkraft erzeugt, die den Gelenkkopf 3 vom Konus 2 abzuschieben trachtet. Sie sollte auch so viel Platz innerhalb der Nut 5 haben, daß im Falle einer späteren Quellung oder sonstigen Volumenvermehrung Ausdehnungsmöglichkeit vorhanden ist und nicht die Gefahr besteht, daß sie mangels Ausdehnungsplatz eine Kraft entwickelt, durch die der Kopf vom Konus wieder abgeschoben werden könnte.

## Patentansprüche

1. Alloplastischer Knochenersatz, insbesondere Gelenkendoprothese, mit mindestens zwei Komponenten (1, 3), die miteinander unmittelbar durch eine Konussteckverbindung (2, 4) verbunden sind, dadurch gekennzeichnet, daß die zusammenwirkenden Konusflächen (2, 4) der Konussteckverbindung nach außen abgedichtet sind.

2. Knochenersatz nach Anspruch 1, dadurch gekennzeichnet, daß einer der beiden Verbindungsteile (1, 3) nahe dem äußeren Ende seiner mit dem anderen Teil zusammenwirkenden Konusfläche (2 bzw. 4) eine Nut (5, 7) enthaltend einen weichelastischen oder weichplastischen Dichtring (6, 9) umfaßt.
